# EUROPÄISCHE PATENTSCHRIFT

(11) **EP 0 358 995 B1**
(45) Veröffentlichungstag und Bekanntmachung des Hinweises auf die Patenterteilung: **22.09.1993**
(21) Anmeldenummer: 89115614.3
(22) Anmeldetag: 24.08.1989
(51) Int. Cl.: A61K 37/52, A61K 37/64

(54) **Verwendung von Transglutaminasen als Immunsuppressiva**
Use of transglutaminases as immunosuppressive agents
Application de transglutaminases comme agents immunosuppressifs

(30) Priorität: 31.08.1988 DE 3829524
(43) Veröffentlichungstag der Anmeldung: 21.03.1990
(73) Patentinhaber: BEHRINGWERKE Aktiengesellschaft, 35001 Marburg (DE)
(72) Erfinder: Stief, Thomas, Dr., E-41007 Sevilla (ES); Heimburger, Norbert, Prof. Dr., D-3550 Marburg (DE); Schorlemmer, Hans Ulrich, Dr., D-3550 Marburg (DE)

(56) Entgegenhaltungen:
- EP-A- 0 086 627
- EP-A- 0 278 416
- EP-A- 0 278 696

## Beschreibung

Die Erfindung betrifft die Verwendung einer Transglutaminase in einem Verfahren zur Herstellung eines Immunsuppressivums.

Makrophagen und polymorphkernige neutrophile Leukozyten (PMN) sind an der Immunantwort des Organismus in wesentlicher Weise beteiligt. Sie verfügen über ein Angriffssystem, das zum einen aus Proteasen und zum anderen aus potenten Oxidantien (N-Chloramine und Sauerstoffradikale) besteht. Über diese Proteasen und Oxidantien beeinflussen und verstärken sie die Immunantwort.

Unter Immunantwort soll verstanden werden die Reaktion immunkompetenter Zellen wie Monozyten/Makrophagen-System auf Entzündungs-, Infektions-, Gewebeumbau- und Gewebereparatur-Reize. Dem Monozyten/Makrophagen-System kommen vielfältige Aufgaben im gesamten Organismus zu, da Blutmonozyten in verschiedenste Gewebe einwandern, um hier nicht zuletzt spezifische immunologische Aufgaben zu übernehmen. So differenzieren sich die Monozyten in der Lunge zu Alveolarmakrophagen, in der Leber zu von Kupfferschen Sternzellen, im Knochen zu Osteoklasten, im zentralen Nervensystem (ZNS) zu Mikrogliazellen, in der Niere zu Mesangialmakrophagen, in der Gelenkschleimhaut zu Synovialisdeckzellen und in den Körperhöhlen zu Pleura- bzw. Peritonealmakrophagen.

Inhibitoren dieser Makrophagen und PMN sind von hohem klinischen Interesse, da eine Vielzahl menschlicher Erkrankungen mit erhöhter Makrophagenaktivität einhergeht.

Es kann Zustände geben, in denen die funktionelle Unterdrückung eines oder mehrerer Kompartimente des Immunsystems wünschenswert ist. Solche Zustände können eine über das Normalmaß hinausschießende Reaktion des Immunsystems (hypererger Zustand) oder eine Reaktion des Immunsystems gegen körpereigenes Gewebe sein.

Substanzen, die in der Lage sind, die biologische Wirkung von Makrophagen zu inhibieren, können immunsuppressiv wirken. Es besteht großer Bedarf nach a) nebenwirkungsarmen und b) hochspezifischen Immunsuppressoren. Die in der Klinik vielfach verwandten Kortikoidabkömmlinge erfüllen weder a) noch b).

Als in in vitro-Testsystemen mit Makrophagen Substanzen auf Sauerstoffradikal-Sekretion inhibierende Wirkung getestet wurden, wurde überraschenderweise gefunden, daß Inhibitoren der Ornithyldecarboxylase besonders vom Typ der Transglutaminasen, vorzugsweise F XIII, sowohl plazentaren als auch plasmatischen Ursprungs, in physiologischen Konzentrationen stark inhibierend auf die auf Sauerstoffradikal-Freisetzung beruhende Chemilumineszenzreaktion von Makrophagen wirkt.

Es wurde weiterhin überraschenderweise gefunden, daß F XIII in vivo an Transplantationsmodellen immunsuppressiv wirkt.

Gegenstand der Erfindung ist daher die Verwendung einer Transglutaminase, vorzugsweise von Faktor XIII, zur Herstellung eines Immunsuppressivums.

Krankheitsbilder, bei denen ein solches Immunsuppressivum angewendet werden kann, sind beispielsweise solche mit chronisch entzündlichem Geschehen wie rheumatoider Arthritis, Morbus Crohn, Colitis ulcerosa, Multiple Sklerose, Guillain-Barre -Syndrom, Psoriasis und anderen Autoimmunerkrankungen, degenerativen Erkrankungen wie Morbus Parkinson, Arteriosklerose, neoplastischen Erkrankungen, hyperergisch-allergischen Erkrankungen, Graft versus Host-Reaktionen, Schocksyndrom wie ARDS (Adult Respiratory Distress Syndrome), Verbrennungskrankheit, Verbrauchskoagulopathie und Sepsis. Auch bei infektiösen Erkrankungen wie AIDS kann einem solchen Mittel Bedeutung zukommen.

Auch nach der Transplantation von körperfremdem Gewebe kommt es zu einer immunologischen Reaktion gegen das als fremd erkannte transplantierte Organ oder gegen den Empfängerorganismus im Sinne einer graft versus host-Reaktion, was zu einer Abstoßung führt. Derartige Abstoßungskrisen können sich auch mit plazentarem Gewebe ergeben, welches vom mütterlichem Organismus z. T. auch als fremd erkannt wird und zu Plazentatrophoblaststörungen und vorzeitiger Plazentalösung führen kann. Es ist daher notwendig, das Immunsystem des Organempfängers oder des Transplantats zu unterdrücken, um das Überleben des Transplantats bzw. des Empfängers zu gewährleisten.

Im Gegensatz zu der in der Klinik eingesetzten Superoxid-dismutase, welche entstandene Superoxid-Ionen zu neutralisieren vermag, verhindert F XIII die Entstehung von Superoxid-Ionen. Transglutaminasen wirken auch bei allergischer Encephalomyelitis (AE), die als Modellerkrankung der menschlichen Krankheit Multiple Sklerose gilt.

Auch neoplastische Erkrankungen können mitbedingt sein durch eine pathologisch gesteigerte Aktivität von Leukozyten. Hierzu zählen z. B. Histocytosis X, Leukämien, vorzugsweise der myeloischen Reihe und bestimmte, sich wie Makrophagen verhaltende Tumoren oder Tumoren, die auf Unterstützung von Makrophagen angewiesen sind (z. B. zur Versorgung des Tumorstroma im Sinne von Angiogenese). Bei diesen neoplastischen Erkrankungen empfiehlt sich daher ebenfalls die erfindungsgemäße immunsupprimierende Therapie.

Über die Norm hinausschießende Reaktionen des Immunsystems und damit einhergehende pathologisch erhöhte Sauerstoffradikal- und/oder Plasminogenaktivator-Freisetzung findet sich auch bei Reperfusionszuständen, d. h. plötzliche Blutdurchströmung eines zuvor minderperfundierten Gewebes, bei hochprozentiger Sauerstoffbeatmung, bei klinischem Einsatz bzw. Vergiftung mit z. B. Paraquat, Behandlung mit Cis-Platin, Adriamycin, Nitrofurantoin, Bleomycin, Streptozotocin und anderen diabetogenen Substanzen, Bestrahlungstherapie und damit einhergehenden Gewebeschäden.

Zu den bekannten Maßnahmen, die für die Unterdrückung des Immunsystems geeignet sind, gehören die Behandlung mit Antikörpern gegen lymphatisches Gewebe, ionisierenden Strahlen und chemischen Substanzen. Eine derartige aggressive Behandlung geht einher mit ausgeprägten Nebenwirkungen: Organotoxizität, Sterilität bei Cytostatika und ionisierenden Strahlen, Hirnödem bei Tranexamsäure, Anti-Antikörperbildung mit Gefahr einer Strahlen und chemischen Substanzen. Eine derartige aggressive Behandlung geht einher mit ausgeprägten Nebenwirkungen: Organotoxizität, Sterilität bei Cytostatika und ionisierenden Strahlen, Hirnödem bei Tranexamsäure, Anti-Antikörperbildung mit Gefahr einer Serumkrankheit bei Behandlung mit Antikörpern.

Eine Verwendung von humanem, physiologischen F XIII hat dagegen keine der Nebenwirkungen und wirkt bereits in niedrigen Dosen wesentlich effektiver. Selbst hohe Konzentrationen von F XIII im menschlichen Plasma scheinen keine nachteiligen Wirkungen auszulösen, so daß sich eine hohe therapeutische Breite dieses Proteins (mindestens bis zu dem 10 fachen der normalen Plasmakonzentration) ergibt.
Eine wirksame Menge an Transglutaminasen zum Inhibieren des Schadens bei gefährdetem Gewebe während der Reperfusion, bei entzündlichen Geschehen, Subarachnoidalblutungen, Autoimmunerkrankungen, degenerativen Erkrankungen, Arteriosklerose, neoplastischen Erkrankungen (wie Leukamien, Histiocytosis X und anderen), hyperergisch-allergischen Erkrankungen, Verbrennungskrankheit, Transplantatunverträglichkeit, vorzeitiger Plazentalösung und Präeklampsie, Schocksyndrom hängt von einer Reihe von Faktoren ab, beispielsweise dem Alter und dem Gewicht des Patienten und dem klinischen Zustand.

Eine wirksame Dosis an einer Transglutaminase, vorzugsweise F XIII beträgt etwa 0,7 - 3000 besonders bevorzugt 7 - 300 E^{*}/kg/24 Stunden, applizierbar i. v. oder lösungsvermittelt i. m.. Transglutaminasen wie F XIII können durch Zusatz von Stabilisatoren wie Albumin, Polygelin oder einer Aminosäure wie Glycin stabilisiert werden (* 1 E entspricht der Menge an F XIII in 1 ml menschlichem Citratplasma).

Transglutaminasen wie F XIII können auch topisch verabreicht werden zur lokalen Therapie und Prophylaxe von Wundheilungsstörungen, Transplantatabstoßungen, asthmoiden Bronchialerkrankungen, Verbrennungen und anderen mit erhöhter Makrophagenaktivität einhergehenden Erkrankungen.

Medizinisch indiziert sind Kombinationen aus Transglutaminasen und einem Fibrinolytikum wie Gewebe-Plasminogenaktivator (t-PA), Urokinase, Streptokinase oder Plasminogen-Streptokinase-Aktivatorkomplex zur Lysetherapie vorzugsweise bei arteriellen Verschlüssen wie bei Myokardinfarkt oder Apoplex. Ein kombinierter Einsatz von Transglutaminasen und Fibrinolytikum ermöglicht die sofortige Inhibition von Gewebeschäden (z. B. Nekrosen) während und nach akuter Reperfusion bedingt durch hyperaktive weiße Blutzellen.

Desweiteren indiziert sind Kombinationen aus Transglutaminasen mit Antithrombin III, humanen plasmatischen oder gentechnologischen Ursprungs zur Verwendung als Immunsuppressivum.

Medizinisch in bevorzugter Weise angezeigt sind Kombinationen aus Transglutaminasen mit Plasminogen-Aktivator-Inhibitor plazentaren Ursprungs (PAI-2) zur Verwendung als Immunsuppressivum. Wird PAI-2 in einer Kombination mit Transglutaminasen verwendet, dann beträgt eine wirksame Dosis an PAI-2 bis 30 000 im allgemeinen 0.7 - 3 000 Urokinase-inhibierende Einheiten/kg/24 Stunden, d.h. 52,5 - 225 000 Einheiten für einen 75 kg schweren Patienten für die systemische Applikation.

Im folgenden wird die Wirkung von Transglutaminasen auf die Immunantwort der Maus und des Menschen in ausgewählten in vivo- und in vitro-Standardtestmethoden, die bekanntermaßen für die Beurteilung von Immunsuppressoren herangezogen werden, beispielhaft erläutert. durchgeführt. Eine Kontrolle mit gereinigtem plasmatischen F XIII führte zu vergleichbaren Ergebnissen.

### Beispiel 1

### Einfluß von Faktor XIII auf die Stimulation humaner Phagozyten in vitro - additiver Effekt von F XIII plus Plasminogen-Aktivator-Inhibitor-2

Es wurden polymorphkernige Granulozyten und mononukleäre Phagozyten aus peripherem Blut gesunder Spender gewonnen und nach Gabe des Präparates auf verschiedene Funktionen geprüft. Als Parameter der Phagozytenfunktion wurden die Chemilumineszenzreaktion und die Sekretion lysosomaler Enzyme untersucht. Gemessen wurde sowohl die Wirkung von Faktor XIII auf die nicht-aktivierten (-IC) als auch auf die durch die durch Immunkomplexe aktivierten Phagozyten (+IC).

Verglichen mit Phagozyten der entsprechenden Kontrollgruppen (unbehandelte Zellen) war die Chemilumineszenzreaktion (Generierung von Sauerstoff-Radikalen) sowohl bei humanen Granulozyten als auch Monozyten (Phagozyten) dosisabhängig signifikant erniedrigt (Tabelle 1).

Diese supprimierende Wirkung von Faktor XIII ist noch deutlicher bei einer Co-Stimulation in vitro mit Immunkomplexen IC (50 µg/ml) ausgeprägt. Humane mononukleäre Phagozyten, die mit der Substanz behandelt werden, zeichnen sich auch besonders dadurch aus, daß bei Ihnen die Sekretion lysosomaler Enzyme deutlich inhibiert ist (Tabelle 2).
Werden 0.05 E F XIII in Kombination mit 0.1 E plazentaren Plasminogen-Aktivator-Inhibitor (PAI-2) unter IC-Stimulation gegeben, ergibt sich eine Inhibition auf unter 40% trotz einer Inhibition von ca. 50% der Einzelsubstanzen, was eine synergistische Wirkungsweise beider Substanzen erklärt.

### Beispiel 2

### Wirkung von Faktor XIII auf die Aktivität von Maus-Peritonealmakrophagen in vivo

Weiblichen NMRI Mäuse (18 - 20 g) wurde Faktor XIII in Konzentrationen von 0,25 - 2,5 E/Tier parenteral verabreicht. Die Kontrollen erhielten gleiche Volumina (0,5 ml) des Lösungsmittels (physiologisch gepufferte Kochsalzlösung, pH 7,2). Zwei Stunden und 24 Stunden später wurden die Mäuse getötet, ihnen die Makrophagen aus der Bauchhöhle entnommen und eine Aktivitätsbestimmung mit Hilfe der Chemilumineszenz, wie im Beispiel 1 beschrieben, durchgeführt. Wie aus Tabelle 3 ersichtlich ist, erniedrigt Faktor XIII die Aktivität von Makrophagen, die Mäusen entnommen wurden, welche zuvor mit dem Präparat behandelt wurden. Die Suppression wurde bei der Chemilumineszenz sowohl nach 2 Stunden als auch nach 24 Stunden nach Applikation der Substanz mit und ohne Zusatz von Immunkomplexen in vitro beobachtet.

### Beispiel 3

### Einfluß von F XIII auf die Überlebenszeit von transplantierter Rattenschwanzhaut

In diesem Experimentalansatz wurden Schwanzhautstückchen von Lewis-Ratten mit einer Größe von etwa 0,5 x 1,0 cm auf den Schwanz von Fischer-Ratten verpflanzt. Die transplantierten Hautstücke werden vom Immunsystem der Empfängertiere als fremd erkannt und abgestoßen. Wie in der Tabelle 4 zu sehen ist, betrug die Transplantatüberlebenszeit im Lewis-Fischer-Rattenmodell in den Kontrollgruppen, die nur mit dem Lösungsmittel behandelt wurden, zwischen 16 und 18 Tagen. Faktor XIII (5 E/Tier) wurden intraperitoneal an 7 aufeinanderfolgenden Tagen entweder beginnend am Tag 1 oder am Tag 10 nach der Transplantation verabreicht. Hierbei zeigte sich überraschend, daß Faktor XIII in der benutzten Konzentration die Überlebenszeiten der Transplantate von 17,0 ± 1,4 auf 24,8 ± 1,9 bzw. 26,8 ± 2,2 erhöhte.

**Tabelle 1**

| Einfluß von Faktor XIII auf den oxydativen Stoffwechsel (Chemilumineszenz) mit und ohne Immunkomplexstimulation (50 µg/ml) in vitro | | | |
|---|---|---|---|
| Zelltyp | Faktor XIII (E/ml) | Chemilumineszenz | |
| | | Integral der RLU/15 min.(x10³) | |
| | | -IC | +IC |
| Human-Monozyten (Phagozyten) 1x10⁶ Zellen | 0 | 1674 ± 245 | 19775 ± 1450 |
| | 2.5 | 252 ± 48 | 947 ± 152 |
| | 1.2 | 384 ± 95 | 1294 ± 58 |
| | 0.6 | 474 ± 61 | 2195 ± 35 |
| | 0.3 | 575 ± 61 | 5195 ± 488 |
| | 0.03 | 637 ± 81 | 6850 ± 354 |
| | 0.01 | 873 + 75 | 10055 + 629 |
| Human-Granulozyten 2x10⁵ Zellen | 0 | 2678 ± 353 | 58650 ± 2124 |
| | 2.5 | 148 ± 24 | 6448 ± 1505 |
| | 1.2 | 342 ± 31 | 9583 ± 1193 |
| | 0.6 | 645 ± 68 | 15733 ± 2035 |
| | 0.3 | 863 ± 51 | 18700 ± 1114 |
| | 0.03 | 1075 ± 134 | 26900 ± 1556 |
| | 0.01 | 1226 ± 186 | 37400 ± 2851 |
| RLU = Relative Lichteinheiten | | | |

**Tabelle 2**

| Einfluß von Faktor XIII auf die Makrophagenaktivität (Human-Monozyten, 3 x 10⁶ Zellen) in vitro. | | |
|---|---|---|
| Faktor XIII (Einheiten/ml) | Enzymfreisetzung (N-Ac-Glu, mU/ml) | |
| | - IC | + IC |
| 0 | 8365 ± 219 | 16205 ± 1703 |
| 2.5 | 2514 ± 574 | 2723 ± 333 |
| 0.6 | 4254 ± 541 | 6428 ± 1112 |
| 0.3 | 6358 ± 632 | 10320 ± 1030 |
| 0.03 | 7703 ± 288 | 13483 ± 794 |

**Tabelle 3**

| Einfluß von Faktor XIII auf die Makrophagenaktivität (1 x 10⁶ Zellen) in vivo. | | | | |
|---|---|---|---|---|
| Faktor XIII E/Tier 1 x i.p. | Chemilumineszenz | | | |
| | Integral der RLU/15 Min. (x 10³) | | | |
| | - IC | | + IC | |
| | 2 Std. | 24 Std. | 2 Std. | 24 Std. |
| 2.5 | 268 ± 54 | 496 ± 79 | - | 4463 ± 567 |
| 1.0 | 512 ± 103 | 959 ± 163 | - | 8485 ± 707 |
| 0.5 | 981 ± 111 | 1290 ± 240 | - | 11300 ± 1424 |
| 0.25 | 1113 ± 67 | 2105 ± 276 | - | 16233 ± 2706 |
| 0 | 2775 ± 375 | 4480 ± 453 | - | 36767 ± 3227 |

**Tabelle 4**

| Effekt von Faktor XIII auf die Hauttransplantation bei Fischer-Ratten | | |
|---|---|---|
| Substanz | Tage der Transplantatabstoßung | Mittelwert (Tage) (x ± s) |
| Kontrolle | 19, 18, 16, 16, 16 | 17,0 ± 1,4 |
| Faktor XIII 5 E/Tier, 7 x i.p. day 1-7 | 22, 27, 24, 26, 25 | 24,8 ± 1,9 |
| Faktor XIII 5 E/Tier, 7 x i.p. day 10-17 | 30, 27, 24, 27, 26 | 26,8 ± 2,2 |

## Patentansprüche (Patentansprüche für folgende(n) Vertragsstaat(en): AT, BE, CH, DE, FR, GB, IT, LU, NL, SE)

1. Verwendung einer Transglutaminase in einem Verfahren zur Herstellung eines Mittels zur Immunsuppression.

2. Verwendung nach Anspruch 1, dadurch gekennzeichnet, daß die Transglutaminase Faktor XIII ist.

3. Verwendung nach Anspruch 1, dadurch gekennzeichnet, daß ein Mittel für die Therapie und Prophylaxe von Transplantatabstoßungskrisen und Plazentatrophoblaststörungen und Graft versus Host-Reaktionen hergestellt wird.

4. Verwendung nach Anspruch 1, dadurch gekennzeichnet, daß ein Mittel für die Therapie von Autoimmunerkrankungen hergestellt wird.

5. Verwendung nach Anspruch 1, dadurch gekennzeichnet, daß ein Mittel für die Therapie von Krankheitsprozessen, die mit erhöhter Sauerstoff-Radikalbildung einhergehen, hergestellt wird.

6. Verwendung nach Anspruch 1, dadurch gekennzeichnet, daß ein Mittel für die Therapie und Prophylaxe von Gewebeschäden während und nach akuter Reperfusion hergestellt wird.

7. Verwendung nach Anspruch 1, dadurch gekennzeichnet, daß ein Mittel zur topischen Anwendung hergestellt wird.

8. Verwendung nach Anspruch 1, dadurch gekennzeichnet, daß ein Mittel für die Unterdrückung des Monocyten-Makrophagen-Systems hergestellt wird.

9. Verwendung nach Anspruch 1, dadurch gekennzeichnet, daß ein Mittel enthaltend 52,5 bis 225 000 Einheiten pro Dosis (75 kg Körpergewicht) Transglutaminase hergestellt wird.

10. Zubereitung enthaltend eine Transglutaminase und einen Plasminogen-Aktivator-Inhibitor, wobei PAI-2 ausgenommen ist.

11. Zubereitung nach Anspruch 10, dadurch gekennzeichnet, daß sie 52,5 bis 225 000 Einheiten pro Dosis (75 kg Körpergewicht) Transglutaminase und 52,5 bis 225 000. Einheiten Plasminogen-Aktivator-Inhibitor pro Dosis enthält.

## Patentansprüche (Patentansprüche für folgende(n) Vertragsstaat(en): ES, GR)

1. Verfahren zur Herstellung eines Arzneimittels zur Immunsuppression, dadurch gekennzeichnet, daß eine Transglutaminase und gegebenenfalls ein Plasminogen-Aktivator-Inhibitor gegebenenfalls unter Zusatz eines Hilfsstoffs oder Stabilisators in eine zur Darreichung geeignete Form bringt.

2. Verfahren nach Anspruch 1, dadurch gekennzeichnet, daß als Transglutaminase Faktor XIII verwendet wird.

3. Verfahren nach Anspruch 1, dadurch gekennzeichnet, daß ein Arzneimittel für die Therapie und Prophylaxe von Transplantatabstoßungskrisen und Plazentatrophoblaststörungen und Graft versus Host-Reaktionen hergestellt wird.

4. Verfahren nach Anspruch 1, dadurch gekennzeichnet, daß ein Arzneimittel für die Therapie von Autoimmunerkrankungen hergestellt wird.

5. Verfahren nach Anspruch 1, dadurch gekennzeichnet, daß ein Arzneimittel für die Therapie von Krankheitsprozessen, die mit erhöhter Sauerstoff-Radikalbildung einhergehen, hergestellt wird.

6. Verfahren nach Anspruch 1, dadurch gekennzeichnet, daß ein Mittel für die Therapie und Prophylaxe von Gewebeschäden während und nach akuter Reperfusion hergestellt wird.

7. Verfahren nach Anspruch 1, dadurch gekennzeichnet, daß ein Mittel zur topischen Anwendung hergestellt wird.

8. Verfahren nach Anspruch 1, dadurch gekennzeichnet, daß ein Mittel für die Unterdrückung des Monocyten-Makrophagen-Systems hergestellt wird.

9. Verfahren nach Anspruch 1, dadurch gekennzeichnet, daß 52,5 bis 225 000 Einheiten pro Dosis (75 kg Körpergewicht) Transglutaminase verwendet werden.

10. Verfahren nach Anspruch 1, dadurch gekennzeichnet, daß ein Plasminogen-Aktivator-Inhibitor, wobei PAI-2 ausgenommen ist, zugesetzt wird.

11. Verfahren nach Anspruch 10, dadurch gekennzeichnet, daß 52,5 bis 225 000 Einheiten pro Dosis (75 kg Körpergewicht) Transglutaminase und 52,5 bis 225 000 Einheiten Plasminogen-Aktivator-Inhibitor pro Dosis zugesetzt werden.

## Claims (Claims for the following Contracting State(s): AT, BE, CH, DE, FR, GB, IT, LU, NL, SE)

1. The use of a transglutaminase in a process for the preparation of an agent for immunosuppression.

2. The use as claimed in claim 1, wherein the transglutaminase is factor XIII.

3. The use as claimed in claim 1, wherein an agent for the therapy and prophylaxis of transplant rejection crises and placenta/trophoblast disturbances and graft-versus-host reactions is prepared.

4. The use as claimed in claim 1, wherein an agent for the therapy of autoimmune diseases is prepared.

5. The use as claimed in claim 1, wherein an agent for the therapy of pathological processes which are associated with increased formation of oxygen radicals is prepared.

6. The use as claimed in claim 1, wherein an agent for the therapy and prophylaxis of tissue damage during and after acute reperfusion is prepared.

7. The use as claimed in claim 1, wherein an agent for topical use is prepared.

8. The use as claimed in claim 1, wherein an agent for suppressing the monocyte/macrophage system is prepared.

9. The use as claimed in claim 1, wherein an agent containing 52.5 to 225,000 units per dose (75 kg body weight) of transglutaminase is prepared.

10. A composition containing a transglutaminase and a plasminogen activator inhibitor, with the exception of PAI-2.

11. A composition as claimed in claim 10, which contains 52.5 to 225,000 units per dose (75 kg body weight) of transglutaminase and 52,5 to 225,000 units of plasminogen activator inhibitor per dose.

## Claims (Claims for the following Contracting State(s): ES, GR)

1. A process for the preparation of a pharmaceutical for immunosuppression, which comprises converting a transglutaminase and, where appropriate, a plasminogen activator inhibitor, where appropriate with the addition of an ancillary substance or stabilizer, into a form suitable for administration.

2. The process as claimed in claim 1, wherein the transglutaminase used is factor XIII.

3. The process as claimed in claim 1, wherein a pharmaceutical for the therapy and prophylaxis of transplant rejection crises and placenta/trophoblast disturbances and graft-versus-host reactions is prepared.

4. The process as claimed in claim 1, wherein a pharmaceutical for the therapy of autoimmune diseases is prepared.

5. The process as claimed in claim 1, wherein a pharmaceutical for the therapy of pathological processes which are associated with increased formation of oxygen radicals is prepared.

6. The process as claimed in claim 1, wherein an agent for the therapy and prophylaxis of tissue damage during and after acute reperfusion is prepared.

7. The process as claimed in claim 1, wherein an agent for topical use is prepared.

8. The process as claimed in claim 1, wherein an agent for suppressing the monocyte/macrophage system is prepared.

9. The process as claimed in claim 1, wherein 52.5 to 225,000 units per dose (75 kg body weight) of transglutaminase are used.

10. The process as claimed in claim 1, wherein a plasminogen activator inhibitor, with the exception of PAI-2, is added.

11. The process as claimed in claim 10, wherein the composition contains 52.5 to 225,000 units per dose (75 kg body weight) of transglutaminase and 52,5 to 225,000 units of plasminogen activator inhibitor per dose.

## Revendications (Revendications pour l'(les) Etat(s) contractant(s) suivant(s): AT, BE, CH, DE, FR, GB, IT, LU, NL, SE)

1. Utilisation d'une transglutaminase dans un procédé pour préparer un agent destiné à l'immunosuppression.

2. Utilisation selon la revendication 1, caractérisée en ce que la transglutaminase est le facteur XIII.

3. Utilisation selon la revendication 1, caractérisée en ce que l'on prépare un agent pour la thérapie et la prophylaxie des crises de rejet de transplant, des perturbations du trophoblaste placentaire et des réactions de type greffe contre receveur.

4. Utilisation selon la revendication 1, caractérisée en ce que l'on prépare un agent pour la thérapie de maladies auto-immunes.

5. Utilisation selon la revendication 1, caractérisée en ce que l'on prépare un agent pour la thérapie des processus pathologiques qui évoluent avec une augmentation de la formation de radicaux oxygène.

6. Utilisation selon la revendication 1, caractérisée en ce que l'on prépare un agent pour la thérapie et la prophylaxie de lésions tissulaires apparaissant au cours de la reperfusion aiguë et après celle-ci.

7. Utilisation selon la revendication 1, caractérisée en ce que l'on prépare un agent destiné à l'utilisation topique.

8. Utilisation selon la revendication 1, caractérisée en ce que l'on prépare un agent pour la suppression du système monocytes-macrophages.

9. Utilisation selon la revendication 1, caractérisée en ce que l'on prépare un agent contenant de 52,5 à 225000 unités de transglutaminase par dose (pour 75 kg de poids corporel).

10. Préparation contenant une transglutaminase et un inhibiteur d'activateur du plasminogène, à l'exclusion de PAI-2.

11. Préparation selon la revendication 10, caractérisée en ce qu'elle contient de 52,5 à 225000 unités de transglutaminase par dose (pour 75 kg de poids corporel) et de 52,5 à 225000 d'unités d'inhibiteur d'activateur du plasminogène par dose.

## Revendications (Revendications pour l'(les) Etat(s) contractant(s) suivant(s): ES, GR)

1. Procédé pour préparer un médicament destiné à l'immunosuppression, caractérisé en ce que l'on met une transglutaminase et, éventuellement, un inhibiteur d'activateur du plasminogène, le cas échéant en ajoutant un adjuvant ou un agent stabilisant, sous une forme galénique appropriée.

2. Procédé selon la revendication 1, caractérisé en ce que l'on utilise le facteur XIII comme transglutaminase.

3. Procédé selon la revendication 1, caractérisé en ce que l'on prépare un médicament pour la thérapie et la prophylaxie des crises de rejet de transplant, des perturbations du trophoblaste placentaire et des réactions de type greffe contre receveur.

4. Procédé selon la revendication 1, caractérisé en ce que l'on prépare un médicament pour la thérapie de maladies auto-immunes.

5. Procédé selon la revendication 1, caractérisé en ce que l'on prépare un médicament pour la thérapie des processus pathologiques qui évoluent avec une augmentation de la formation de radicaux oxygène.

6. Procédé selon la revendication 1, caractérisé en ce que l'on prépare un agent pour la thérapie et la prophylaxie de lésions tissulaires apparaissant au cours de la reperfusion aiguë et après celle-ci.

7. Procédé selon la revendication 1, caractérisé en ce que l'on prépare un agent destiné à l'utilisation topique.

8. Procédé selon la revendication 1, caractérisé en ce que l'on prépare un agent pour la suppression du système monocytes-macrophages.

9. Procédé selon la revendication 1, caractérisé en ce que l'on utilise de 52,5 à 225000 unités de transglutaminase par dose (pour 75 kg de poids corporel).

10. Procédé selon la revendication 1, caractérisé en ce que l'on ajoute un inhibiteur d'activateur du plasminogène, à l'exclusion de PAI-2.

11. Procédé selon la revendication 10, caractérisé en ce que l'on utilise de 52,5 à 225000 unités de transglutaminase par dose (pour 75 kg de poids corporel) et de 52,5 à 225000 unités d'inhibiteur d'activateur du plasminogène par dose.
